# EUROPEAN PATENT SPECIFICATION

(11) **EP 4 342 525 B1**
(45) Date of publication and mention of the grant of the patent: **06.05.2026**
(21) Application number: 23198612.6
(22) Date of filing: 20.09.2023
(51) Int. Cl.: A61N 5/06, A61F 9/02

(54) **A DEVICE FOR IMPROVING THE CONDITIONS OF AN EYE**
VORRICHTUNG ZUR VERBESSERUNG VON AUGENLEIDEN
DISPOSITIF POUR L'AMÉLIORATION DES CONDITIONS D'UN OEIL

(30) Priority: 20.09.2022 US 202263408113 P
(43) Date of publication of application: 27.03.2024
(73) Proprietor: The Hong Kong University of Science and Technology, Kowloon, Hong Kong (CN)
(72) Inventor: LAM, David Chuen Chun, Hong Kong (CN); LEUNG, Yun Yuen, Hong Kong (CN)
(74) Representative: Cohausz & Florack

(56) References cited:
- US-A1- 2015 366 706
- US-A1- 2018 207 029
- US-A1- 2020 360 723
- US-A1- 2021 121 327
- US-A1- 2022 117 784
- US-B1- 6 364 875

## Description

### TECHNICAL FIELD

The present disclosure belongs to the technical field of treatment of condition, and specifically relates to a device for improving a state of an eye in a subject and to reduce the risk of suffering from glaucoma.

### BACKGROUND

Glaucoma is an eye disease associated with damage to the optic nerve that can lead to vision loss. The risk of glaucoma increases with age and is related to ethnic or family history. However, there is no authorized preventive measure for patients in high-risk groups. High-risk individuals must rely upon regular eye exams, and treatments can only be started after diagnosis.

Damage to the optic nerve can be caused by excessive intraocular pressure (IOP), resulting in over critical loading stresses in the optic nerve tissues. Lowering the ocular stresses is a crucial approach to slow down glaucoma progression and to inhibit glaucoma from developing in high-risk groups. The ocular stresses are associated with the biomechanical properties of the ocular tissue. The biomechanical property of the ocular tissue is an individual factor and is known to be related to aging. Ocular tissues with higher compliance are desired. High compliance tissues can have higher non-damaging deformation when response to gradual loading from IOP and result in lower accumulation of stresses.

The regulation of the aqueous humor is also essential for IOP control. IOP is primarily controlled by the formation and the drainage of aqueous humor inside an eyeball. The drainage system of an eye is associated with the venous system connected to the Schlemm's canal. Maintaining a healthy condition of the blood circulation and the tissues supporting drainage is essential to inhibit aqueous humor accumulation that can lead to significant elevation of IOP.

High IOP is not an inevitable feature of developing glaucoma. The biomechanical strength of the tissue associated to the optic nerve is also important to resist the damage. The exact mechanisms of deterioration and damage of the ocular tissues are unknown. But the poor vascular circulation of the peripapillary vessel and ineffective tissue cell metabolism are believed to be factors that affect the health of the optic nerve.

Related art technologies have not fully addressed the above concerns. United States Patent 6,364,875 to Stanley, III teaches the combined effect of elevated temperature and stretching forces lead to permanent residual strains, reshaping the cornea. United States Patent 10,881,550 to Tedford et al. teaches multi-wavelength low level light therapy wherein two or more doses of light are delivered in a coordinated fashion to influence a desired target cell functionality. United States Patent Application Publication 2022/0168136 to BADAWI et al. teaches a patch or strip affixed to the skin of the upper and/or lower eyelids to deliver heat to the one or more meibomian glands contained within the underlying skin for dry eye treatment. Medina (Eye (2017) 31, 1621-1627; doi:10.1038/eye.2017.123) teaches that Pneumatic Keratology can be used to alter the cornea by non-invasive means. A vacuum chamber with radial openings alters the collagen fibers in the stroma and flattens the cornea. Jang et al. (Sci. Adv. 2021; 7 : eabf7194 31 March 2021) teaches a soft, smart contact lens and a skin-attachable therapeutic device for wireless monitoring and therapy of chronic ocular surface inflammation (OSI). US 2020/360723A1 discloses an apparatus for improving a condition of an eye in a subject comprising a heat module configured and adapted to warm a cornea, a peripapillary sclera, or an extraocular muscle of the eye to a threshold value that is above a skin surface temperature of the subject; a pressure control module configured and adapted to stretch the cornea, the peripapillary sclera, or the extraocular muscle of the eye of the eye to an extent sufficient to measurably change a periocular pressure of the eye, or an intraocular pressure of the eye, or both; a radiation module configured and adapted to irradiate one or more tissues of an eye with an infra-red radiation; and a controller 16 operably connected to the heat pad module, the pressure control module, and the radiation module, the apparatus uses the simultaneous combination of heat and suction to permanently stretch some of the collagen fibrils within the cornea, resulting in a change in the shape of the cornea and thus the focus of the eye.

### SUMMARY

Embodiments of the subject invention provide devices to improve the states of an eye and to reduce the risk of suffering from glaucoma.

Damage to the optic nerve can be caused by excessive intraocular pressure (IOP), resulting in over critical loading stresses in the optic nerve tissues. Lowering the ocular stresses is a crucial approach to slow down glaucoma progression and to inhibit glaucoma from developing in high-risk groups. The ocular stresses are associated with the biomechanical properties of the ocular tissue. The biomechanical property of the ocular tissue is an individual factor and is known to be related to aging. Ocular tissues with higher compliance are desired. High compliance tissues can have higher non-damaging deformation when response to gradual loading from IOP and result in lower accumulation of stresses. Embodiments of the subject invention advantageously provide warming of the ocular tissues to increase compliance and improve condition of the eye.

The regulation of the aqueous humor is also essential for IOP control. IOP is primarily controlled by the formation and the drainage of aqueous humor inside an eyeball. The drainage system of an eye is associated with the venous system connected to the Schlemm's canal. Maintaining a healthy condition of the blood circulation and the tissues supporting drainage is essential to inhibit aqueous humor accumulation that can lead to significant elevation of IOP.

High IOP is not an inevitable feature of developing glaucoma. The biomechanical strength of the tissue associated to the optic nerve is also important to resist the damage. The exact mechanisms of deterioration and damage of the ocular tissues are unknown. But the poor vascular circulation of the peripapillary vessel and ineffective tissue cell metabolism are believed to be factors that affect the health of the optic nerve.

Aspect of the present invention and claimed herein is a device for improving a state of an eye in a subject, the device comprising a pressure control module configured and adapted to stretch the cornea, the peripapillary sclera, or the extraocular muscle of the eye of the eye to an extent sufficient to measurably change a periocular pressure of the eye, or an intraocular pressure of the eye, or both; wherein the device further comprises: a goggle carrier; a heat pad module configured and adapted to warm a cornea, a peripapillary sclera, or an extraocular muscle of the eye to a threshold value that is above a skin surface temperature of the subject;a radiation module configured and adapted to irradiate one or more tissues of an optic nerve head and the peripapillary sclera in a posterior chamber of the eye with an infra-red radiation having a peak wavelength between 780 nm and 950 nm and with a visible light radiation having a peak wavelength between 550 to 700 nm; and a controller operably connected to the heat pad module, the pressure control module, and the radiation module, the controller configured and adapted to provide heat, pressure, and radiation until reaching a desired state comprising a measureable change in at least one of reduction of an intraocular pressure (IOP) of the eye, an increase of a vascular circulation of a peripapillary vessel of the eye, and an increase of a metabolic rate of one or more groups of cells of the eye.

Other aspects of the present invention and claimed herein are as defined in claims 2-15.

### BRIEF DESCRIPTION OF DRAWINGS

The patent or application file contains at least one drawing executed in color. Copies of this patent or patent application publication with color drawing(s) will be provided by the Office upon request and payment of the necessary fee.
FIG. 1A is a flowchart showing a method [100] to modify the condition of an eye.
FIG. 1B is a second flowchart showing method [200], adding irradiation and other steps in a method similar to method [100].
FIG. 1C is a third flowchart showing method [300], a modified expansion of method [100].
FIGs. 2A - 2B are charts showing measurements of intraocular pressure (IOP) in right (FIG. 2A) and left (FIG. 2B) eyes, respectively, over 11 days of treatment.
FIGs. 3A - 3B are charts showing measurements of intraocular pressure (IOP) of one subject in left (FIG. 3A) and right (FIG. 3B) eyes, respectively, over 2 weeks of baseline measurement plus10 weeks of treatment under method [300].
FIGs. 3C - 3D are charts showing measurements of intraocular pressure (IOP) of another subject in left (FIG. 3C) and right (FIG. 3D) eyes, respectively, over 2 weeks of baseline measurement plus 15 weeks of treatment under method [300].
FIGs. 4A-4B illustrate the experimental setup according to an embodiment of the subject invention, as used to collect the IOP data in Figures 2A-2B and Figures 3A-3D.
FIGs. 5A-5B illustrate the first prototype device according to an embodiment of the subject invention.
FIGs. 6A and 6E illustrate the overview of the second prototype device according to an embodiment of the subject invention.
FIGs. 6B and 6F illustrate the heat pad of the second prototype device according to an embodiment of the subject invention.
FIG. 6C illustrates the modular components of the second prototype device according to an embodiment of the subject invention.
FIG. 6D illustrates the second prototype device during operation according to an embodiment of the subject invention.
FIG. 7A illustrates the irradiation spectra centralized at 590nm wavelength, e.g., for use with Step [251] according to an embodiment of the subject invention.
FIG. 7B illustrates the irradiation spectra centralized at 830nm wavelength, e.g., for use with Step [252] according to an embodiment of the subject invention.

### DETAIL DESCRIPTION OF EMBODIMENTS

According to one or more embodiments of the subject invention, there is provided a system to modify the condition of an eye involving increasing compliance (e.g., in a patient experiencing reduced compliance as a complication of glaucoma) by warm compress of the eye by putting a temperature-controlled heat pad in contact with the eye lid and the periocular region to warm up the tissues of the eye.

As used herein, "subject", "patient", "host" or "organism" refers to any member of the phylum Chordata, more preferably any member of the subphylum vertebrata, or most preferably, any member of the class Mammalia, including, without limitation, humans and other primates, including non-human primates such as rhesus macaques, chimpanzees and other monkey and ape species; livestock, such as cattle, sheep, pigs, goats and horses; domestic mammals, such as dogs and cats; laboratory animals, including rabbits, mice, rats and guinea pigs; birds, including domestic, wild, and game birds, such as chickens, turkeys, ducks, and geese. The term does not denote a particular age or gender. Thus, adult, young, and new-born individuals are intended to be covered as well as male and female subjects. In some embodiments, the subject is a non-human subject. In some embodiments, the subject is a human subject. In some embodiments, the subject can be any living creature having eyes suitable for application of the systems and methods described herein.

According to one or more embodiments of the subject invention, there is provided a system to modify the condition of an eye involving increasing compliance (e.g., in a patient experiencing reduced compliance as a complication of glaucoma) by warm compress of the eye by putting a temperature-controlled heat pad in contact with the periocular region to warm up the tissues of the eye.

According to one or more embodiments of the subject invention, there is provided a system configured to perform a method to modify the condition of an eye and to reduce the risk of suffering from glaucoma, the method involving increasing compliance (e.g., in a patient experiencing reduced compliance as a complication of glaucoma) by stretching the tissue of the cornea and the associated scleral region by applying a static pressure on the periocular region through a goggle device with a pressure-controlled chamber covering the periocular region.

According to one or more embodiments of the subject invention, there is provided a system to modify the condition of an eye and to reduce the risk of suffering from glaucoma involving increasing compliance (e.g., in a patient experiencing reduced compliance as a complication of glaucoma) by stretching the tissue of the cornea and the associated scleral region by applying a combination of static and dynamic pressure on the periocular region through a goggle device with a pressure-controlled chamber covering the periocular region.

According to one or more embodiments of the subject invention, there is provided a system to modify the condition of an eye and to reduce the risk of suffering from glaucoma involving promoting circulation within the blood system connected to the Schlemm's canal by warm compress of the eye by putting a temperature-controlled heat pad in contact with the eye lid to promote the vasodilation of the blood vessels at the eye.

According to one or more embodiments of the subject invention, there is provided a system to modify the condition of an eye and to reduce the risk of suffering from glaucoma involving promoting circulation with the blood system connected to the Schlemm's canal by warm compress of the eye by putting a temperature-controlled heat pad in contact with the periocular region to promote the vasodilation of the blood vessels at the eye.

According to one or more embodiments of the subject invention, there is provided a system to modify the condition of an eye and to reduce the risk of suffering from glaucoma involving promoting the circulation with the peripapillary vessel by irradiating the anterior and posterior chamber of the eye with infra-red radiation with a single wavelength (e.g., between 780 to 950 nm) or with a combination of peak wavelengths (e.g., between 780 to 950 nm.)

According to one or more embodiments of the subject invention, there is provided a system to modify the condition of an eye and to reduce the risk of suffering from glaucoma involving promoting circulation with the peripapillary vessel by irradiating the anterior and posterior chamber of the eye with infra-red radiation with a single wavelength (e.g., between 3000 to 12000 nm) or with a combination of wavelengths between 3000 to 12000 nm.

According to one or more embodiments of the subject invention, there is provided a system to modify the condition of an eye and to reduce the risk of suffering from glaucoma involving promoting tissue cell metabolism by irradiating the anterior and posterior chamber of the eye with visible radiation with single wavelength (e.g., between 550 to 700 nm) or a combination of peak wavelength between 550 to 700nm.

According to one or more embodiments of the subject invention, any of the aforementioned systems and/or methods can be applied alone or in combination with each other and with other systems and/or methods. Such systems and methods can include therapeutic and diagnostic systems, and treatment for certain conditions or diseases. The scope of the invention is defined by the claims. Methods, in particular the medical methods, are described herein for illustrative purposes and are not part of the invention. Claimed herein is a device for improving a state of an eye in a subject, the device comprising a pressure control module configured and adapted to stretch the cornea, the peripapillary sclera, or the extraocular muscle of the eye of the eye to an extent sufficient to measurably change a periocular pressure of the eye, or an intraocular pressure of the eye, or both; wherein the device further comprises: a goggle carrier; a heat pad module configured and adapted to warm a cornea, a peripapillary sclera, or an extraocular muscle of the eye to a threshold value that is above a skin surface temperature of the subject; a radiation module configured and adapted to irradiate one or more tissues of an optic nerve head and the peripapillary sclera in a posterior chamber of the eye with an infra-red radiation having a peak wavelength between 780 nm and 950 nm and with a visible light radiation having a peak wavelength between 550 to 700 nm; and a controller operably connected to the heat pad module, the pressure control module, and the radiation module, the controller configured and adapted to provide heat, pressure, and radiation until reaching a desired state comprising a measureable change in at least one of reduction of an intraocular pressure (IOP) of the eye, an increase of a vascular circulation of a peripapillary vessel of the eye, and an increase of a metabolic rate of one or more groups of cells of the eye.

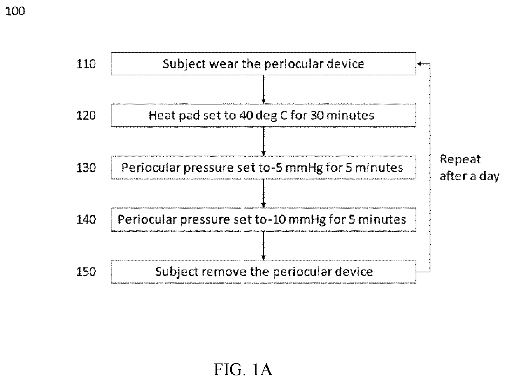

Turning now to the Figures, the embodiment shown in Figure 1A is an example of a method [100] to modify the eye condition of a patient. The subject can wear, apply, or put on a device in Step [110] that has a temperature-controlled eye pad (e.g., a heat pad) and a pressure chamber covering the periocular region. The heat pad that contacts the eye lid and the periocular skin is set to 40 deg C for 30 minutes of warm compress in Step [120]. After the warm compress, the pressure level of the pressure chamber that covers the periocular region is set to -5 mmHg for 5 minutes, stretching of the ocular tissue in Step [130]. Then the pressure level of the pressure chamber that covers the periocular region is set to -10 mmHg for 5 minutes of further stretching of the ocular tissues in Step [140]. After the execution, the subject can remove the device in Step [150] and repeat the method after a sufficient rest period (e.g., one day.)

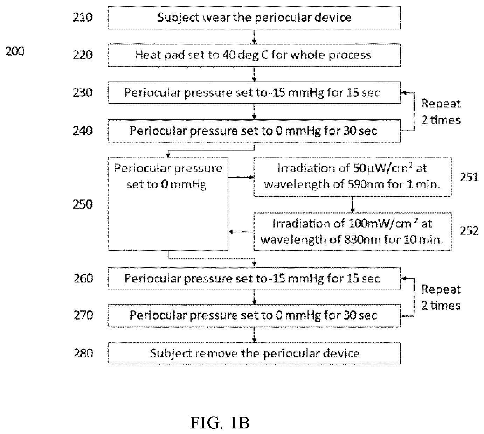

Figure 1B is a second example of a method [200] to modify the eye condition of a patient. The subject can wear a device in Step [210] that has a temperature-controlled eye pad (e.g., a heat pad) and a pressure chamber covering the periocular region. The heat pad that contacts the eye lid and the periocular skin is set to 40 deg C of warm compress for the whole process in Step [220]. During the warm compress, the pressure level of the pressure chamber that covers the periocular region is set to -15 mmHg for 15 seconds, stretching of the ocular tissue in Step [230]. Then the pressure level of the pressure chamber is returned to 0 mmHg for 30 seconds in Step [240]. This pressure cycle in Step [230] to Step [240] between -15 mmHg to 0 mmHg is repeated twice. After the stretching of the ocular tissue, the irradiation of 50µW/cm2 at the 590nm wavelength is applied for 1 minute in Step [251] to activate the epidermis and dermis. Then the irradiation of 100mW/cm2 at the 830nm wavelength is applied for 10 minutes in Step [252] to penetrate in the eyelids for healing and tissue rejuvenation. After the irradiation, the pressure level of the pressure chamber that covers the periocular region is set to -15 mmHg for 15 seconds, to create stretching of the ocular tissue in Step [260]. Then the pressure level of the pressure chamber is returned to 0 mmHg for 30 seconds in Step [270]. This pressure cycle Step [260] to Step [270] between -15 mmHg to 0 mmHg is repeated twice. After the execution, the subject can remove the device in Step [280] and repeat the method after a sufficient rest period (e.g., one day.)

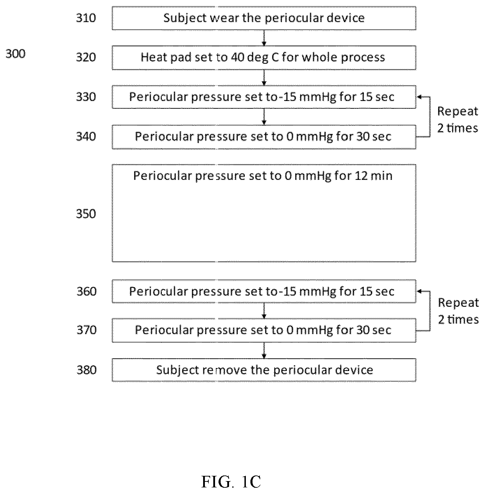

Figure 1C is a third example of a method [300] to modify the eye condition of a patient. The subject can wear a device in Step [310] that has a temperature-controlled eye pad (heat pad) and a pressure chamber covering the periocular region. The heat pad that contacts the eyelid and the periocular skin is set to 40 deg C of warm compress during the whole process in Step [320]. During the warm compress, the pressure level of the pressure chamber that covers the periocular region is set to -15 mmHg for 15 seconds, stretching of the ocular tissue in Step [330]. Then the pressure level of the pressure chamber is returned to 0 mmHg for 30 seconds in Step [340]. This pressure cycle [Step 330 to 340] between -15 mmHg to 0 mmHg is repeated twice. After the stretching of the ocular tissue, the eyes are warm compress for 12 minutes [Step 350]. After the warm compress, the pressure level of the pressure chamber that covers the periocular region is set to -15 mmHg for 15 seconds, stretching of the ocular tissue in Step [360]. Then the pressure level of the pressure chamber is returned to 0 mmHg for 30 seconds in Step [370]. This pressure cycle [Step 260 to 270] between -15 mmHg to 0 mmHg is repeated twice. After the execution, the subject can remove the device in Step [280] and repeat the method after a sufficient rest period (e.g., one day.)

In embodiments of the subject invention, including but not limited to those illustrated in Figures 1A-1C above, additional values and ranges are contemplated. For example, heat can be applied through a heat pad or other means at temperatures from 37.8 oC to 45 oC, including increments, combinations, and ranges thereof. Gauge pressures can be applied from -30 mmHg to 0 mmHg, including increments, combinations, and ranges thereof. Irradiation can be applied in visible, infra-red, or other spectra known in the art. Irradiation can be applied at an intensity from 5µW/cm2 to 1W/cm2, including increments, combinations, and ranges thereof. Irradiation can be applied at wavelengths from 550nm to 12,000nm, including increments, combinations, and ranges thereof. Pressures, irradiation, and/or temperatures can be applied simultaneously, alternatingly, or in series from 1 to 10 times or more, including increments, combinations, and ranges thereof. Pressures, irradiation and/or temperatures can be applied from 1 second to 1 hour in duration, including increments, combinations, and ranges thereof. Appropriate values, application times, combinations, sequences, and other variables can be selected from the provided ranges to achieve desired effects without undue risk to the patient.

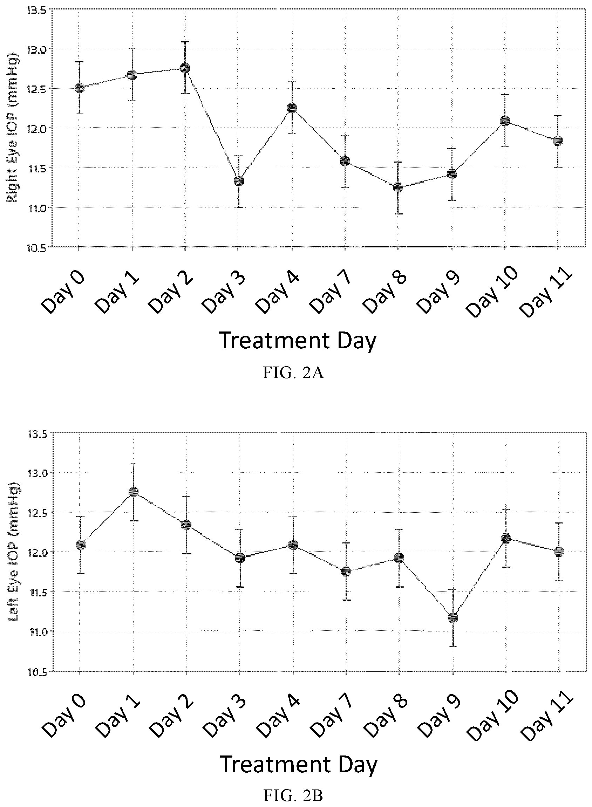

Figures 2A-2B show a set of IOP measurement results from a subject after applying treatment according to the example described by method [100]. The IOP of both left eye and right eye was measured using an air-puff tonometer every time before applying the method. There is a decreasing trend of IOP for both eyes after applying the method [100].

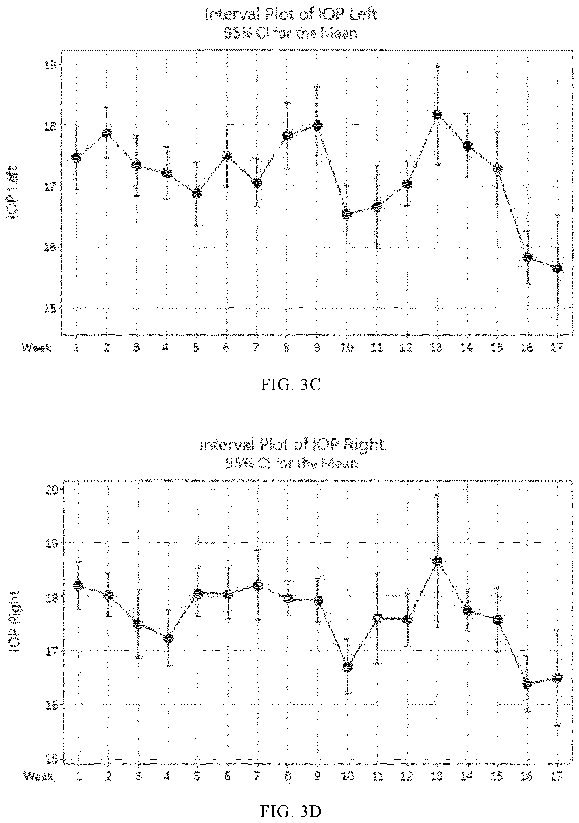

Figures 3C - 3D are charts showing measurements of intraocular pressure (IOP) of another subject in left (FIG. 3C) and right (FIG. 3D) eyes, respectively, over 15 weeks of treatment according to an example of the subject invention under method [300].

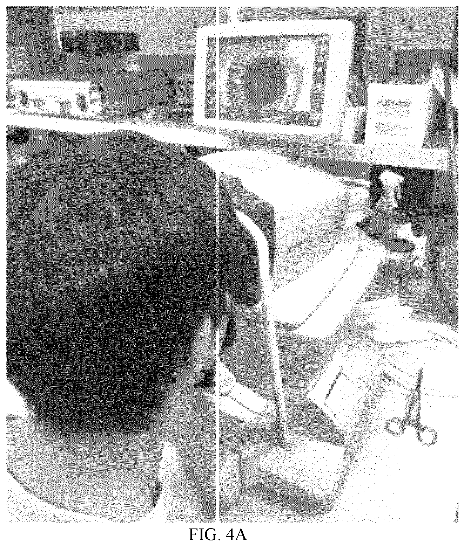

Figures 4A-4B illustrate the measurement of intraocular pressure using an air puff tonometer (Topcon CT-1P). The subject was assigned to use the device according to method [100]. The intraocular pressure of the subject was measured the following day after using the device. Before the measurement, the subject was requested to rest for 5 minutes to calm down. The measurement was taken 3 times for each eye, and the data in FIG 2A and 2B show the mean and the 95% confident range.

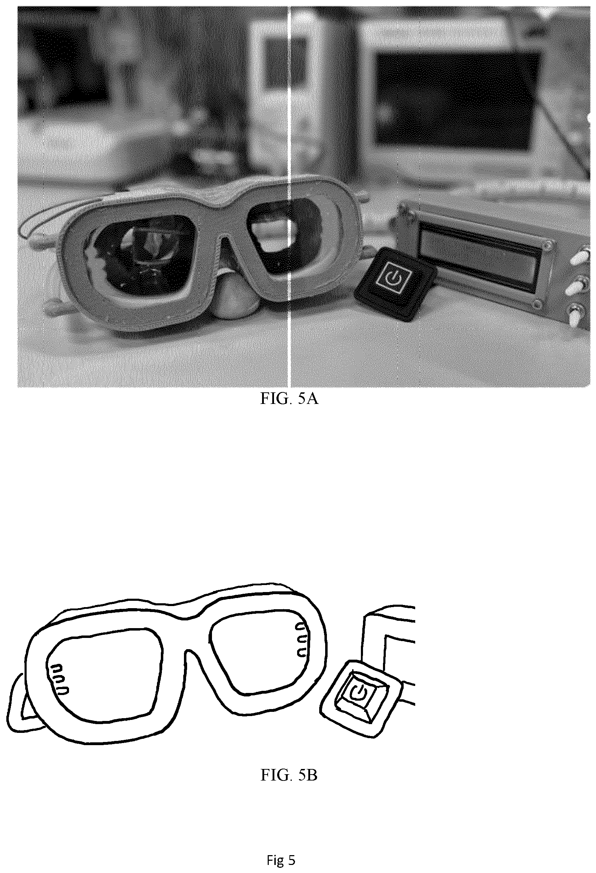

Figures 5A-5B illustrate the first prototype of the device. The device consists of a goggle style carrier containing the heat pad module, the pressure control module, and the radiation module. The goggle carrier is connected to a controller through electrical wires and soft piping. The controller contains an electronic circuit, a pumping system, and a power module. The electronics circuit is for monitoring and control of the process. The pumping system is driven to adjust the air flow to control the goggle carrier pressure. The power module is for battery charging and providing power for the device operation.

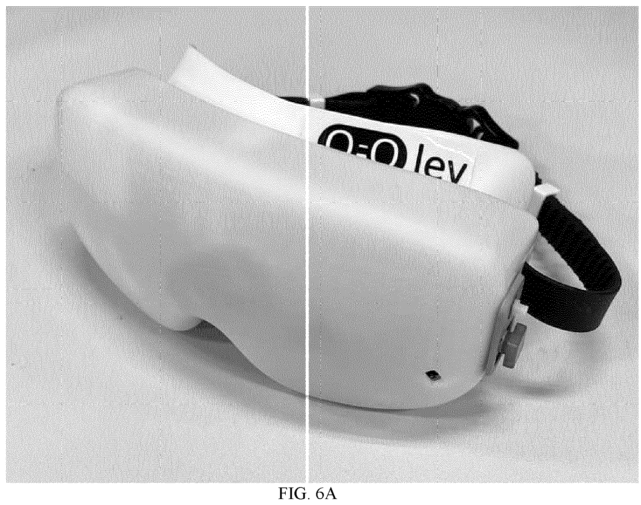

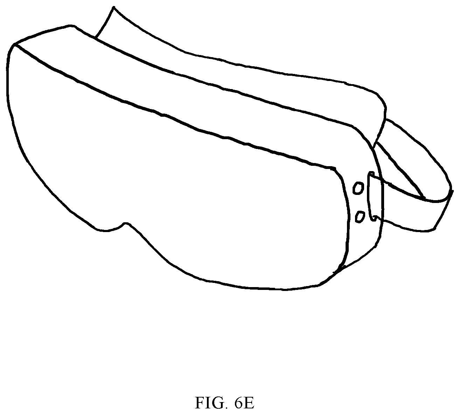

Figures 6A and 6E illustrate the second prototype of the device. The device consists of a goggle style carrier containing the heat pad module, the pressure control module, and the radiation module. All the modules are embedded into the carrier.

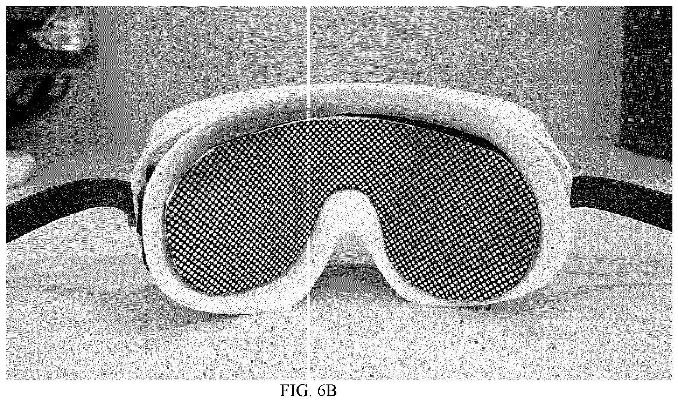

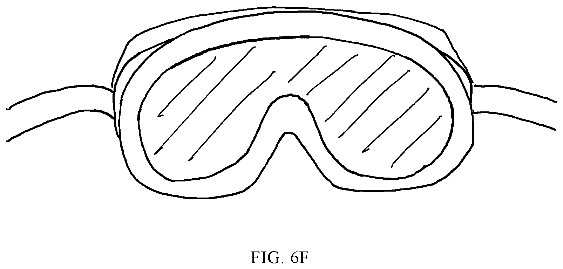

Figures 6B and 6F illustrate the heat pad of the second prototype of the device viewing from the back.

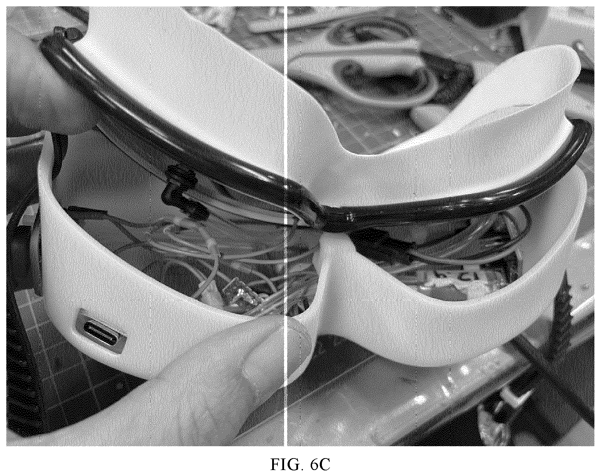

Figure 6C illustrates the modular components of the second prototype of the device embedded inside the casing.

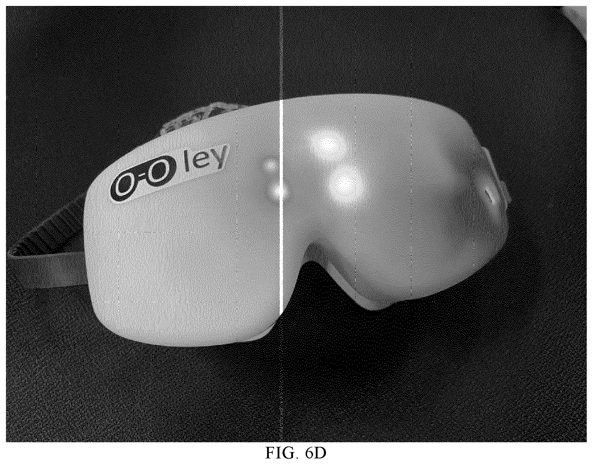

Figure 6D illustrates the second prototype of the device during operation with signal indicator light.

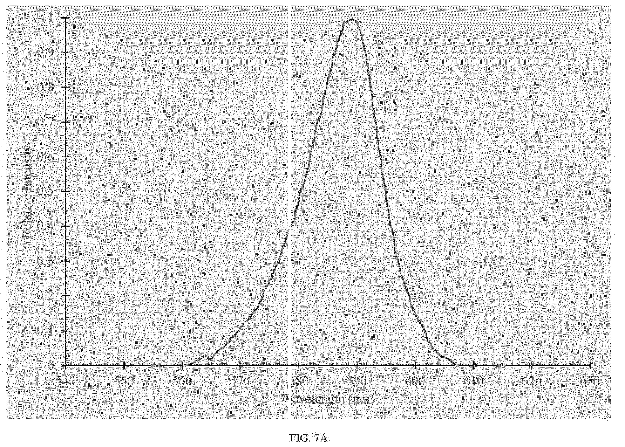

Figure 7A illustrates the irradiation spectra centralized at 590nm wavelength for use with [Step 251].

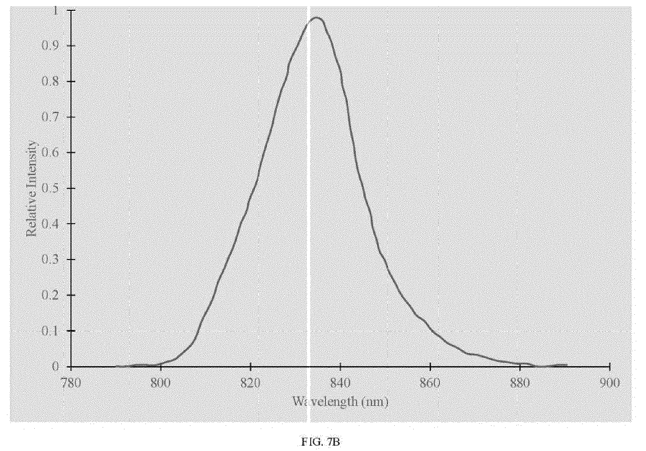

Figure 7B illustrates the irradiation spectra centralized at 830nm wavelength for use with [Step 252].

In order that the present disclosure may be more readily understood, certain terms are defined below, and throughout the detailed description, to provide guidance as to their meaning as used herein.

As used herein, the terms "a," "an," "the" and similar terms used in the context of the present invention are to be construed to cover both the singular and plural unless otherwise indicated herein or clearly contradicted by the context. Thus, for example, reference to "an arm" or "a hole" should be construed to cover or encompass both a singular arm or a singular hole and a plurality of arms and a plurality of holes, unless indicated otherwise or clearly contradicted by the context.

As used herein, the terms "about" and "approximately" shall generally mean an acceptable degree of error for the quantity measured given the nature or precision of the measurements. Exemplary degrees of error are within 20 percent (%), typically, within 10%, and more typically, within 5% of a given value or range of values.

As used herein, the term "and/or" should be understood to mean "either or both" of the features so conjoined, i.e., elements that are conjunctively present in some cases and disjunctively present in other cases.

As used herein, the terms "comprising", "consisting of" and "consisting essentially of" are defined according to their standard meaning. The terms may be substituted for one another herein in order to attach the specific meaning associated with each term.

As used herein, the term "or" should be understood to have the same meaning as "and/or" as defined above. For example, when separating a listing of items, "and/or" or "or" shall be interpreted as being inclusive, i.e., the inclusion of at least one, but also including more than one, of a number of items, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

The invention may be better understood by reference to certain illustrative examples, including but not limited to the following:

Claimed herein is a device for improving a state of an eye in a subject, the device comprising:
a goggle carrier;
a heat pad module configured and adapted to warm a cornea, a peripapillary sclera, or an extraocular muscle of the eye to a threshold value that is above a skin surface temperature of the subject;
a pressure control module configured and adapted to stretch the cornea, the peripapillary sclera, or the extraocular muscle of the eye of the eye to an extent sufficient to measurably change a periocular pressure of the eye, or an intraocular pressure of the eye, or both;
a radiation module configured and adapted to irradiate one or more tissues of an optic nerve head and the peripapillary sclera in a posterior chamber of the eye with an infra-red radiation having a peak wavelength between 780 nm and 950 nm and with a visible light radiation having a peak wavelength between 550 to 700 nm; and
a controller operably connected to the heat pad module, the pressure control module, and the radiation module, the controller configured and adapted to provide heat, pressure, and radiation until reaching a desired state comprising a measureable change in at least one of reduction of an intraocular pressure (IOP) of the eye, increase of a biomechanical compliance of the cornea of the eye, an increase of a vascular circulation connected to a Schlemm's canal of the eye, an increase of a vascular circulation of a peripapillary vessel of the eye, and an increase of a metabolic rate of one or more groups of cells of the eye.

In one example, the goggle carrier is covering a periocular region of the eye.

In one example, the eye is a first eye and the goggle carrier is covering the periocular region the first eye and also covering a periocular region of a second eye.

In one example, the heat pad module comprises a heating element layer, a thermal insulation layer, and a soft contact layer.

In one example, the heating element layer consists of a resistive heating strip embedded in a flexible printed circuit.

In one example, the resistive heating strip embedded in the flexible printed circuit is powered by a pulse width modulated input voltage regulated by the controller.

In one example, the thermal insulation layer is in contact with a first side of the heating element layer.

In one example, the thermal insulation layer comprises a flexible material that conforms to a curved surface on or around the eye.

In one example, the soft contact layer is in contact with a second side of the heating element layer.

In one example, the soft contact layer is configured and adapted for contact with a layer of skin on an eyelid covering the eye.

In one example, the pressure control module comprises a pump.

In one example, the pressure control module comprises a first cannula fluidly coupling the pump to a cavity of the goggle carrier adjacent the periocular region.

In one example, the pressure control module comprises a second cannula fluidly coupling the cavity to an area of ambient pressure.

In one example, the second cannula comprises a check valve configured and adapted to allow fluid flow from the area of ambient pressure into the cavity while inhibiting fluid flow from the cavity into the area of ambient pressure.

In one example, the check valve is configured and adapted to open when the gauge pressure in the cavity drops below a threshold pressure.

In one example, the threshold pressure is equal or lower than -10 mmHg.

In one example, the radiation module comprises a light source configured and adpated to supply the infra-red radiation and the visible light radiation to irradiate an anterior chamber of the eye, the posterior chamber of the eye, and an eyelid of the eye.

In one example, the light source comprises a visible light emitting element operably connected to a first optical device.

In one example, the light source comprises an infra-red light emitting element operably connected to a second optical device.

In one example, the visible light emitting element is powered by a first pulse width modulated input current regulated by the controller.

In one example, the infra-red light emitting element is powered by a second pulse width modulated input current regulated by the controller.

In one example, the operable connection between the infra-red light emitting element and the second optical device is configured and adapted to maximize a first radiation luminous intensity distribution output of the infra-red radiation to one or more of the anterior chamber of the eye, the posterior chamber of the eye, and the eyelid of the eye.

In one example, the controller is configured and adapted to operably couple the regulation of the first pulse width modulated input current and the regulation of the second pulse width modulated input current to deliver a specified total radiation luminous intensity distribution output of the combined visible light radiation and infra-red radiation to one or more of the anterior chamber of the eye, the posterior chamber of the eye, and the eyelid of the eye.

In one example, the second optical devices comprises or consists of a light guide.

In one example, the second optical devices comprises or consists of an optical lens.

In one example, the first optical devices comprises or consists of a light guide.

In one example, the first optical devices comprises or consists of an optical lens.

In one example, stretching tissues of the anterior region of the eye comprises stretching the tissue with a blow nose action by the subject.

In one example, a goggle carrier is covering the periocular region with one eye.

In one example, a goggle carrier is covering the periocular region with both eyes.

In one example, a heat pad module consists of a heating element layer, a thermal insulation layer, and a soft contact layer.

In one example, a heating element layer consists of a resistive heating strip embedded in a flexible printed circuit.

In one example, a resistive heating strip embedded in a flexible printed circuit is powered by a pulse width modulated input voltage regulated by the controller.

In one example, a thermal insulation layer on one side is in contact with the heating element layer.

In one example, a thermal insulation layer consists of flexible material that conform with a curve surface.

In one example, a soft contact layer on one side is in contact with the heating element layer.

In one example, a soft contact layer on one side is in contact with the skin on eyelid.

In one example, a pressure control module consists of a pump.

In one example, a pressure control module further consists of a first group of one or more cannulas configured for fluidly coupling the pump to a cavity of the goggle carrier at the periocular region.

In one example, a pressure control module further consists of a second group of one or more cannulas configured for fluidly coupling the cavity of the goggle carrier at the periocular region to the ambient.

In one example, the second group of one or more cannulas comprises a check valve that only allows fluid flowing from the ambient into the cavity of the goggle carrier at the periocular region.

In one example, the check valve opens when the gauge pressure in the cavity of the goggle carrier at the periocular region drops below a threshold pressure.

In one example, a threshold pressure is equal or lower than -10mmHg.

In one example, a radiation module consists of a light source configured for directing the radiation on an anterior chamber, a posterior chamber, and an eyelid of an eye.

In one example, a light source consists of one or plurality of visible light emitting elements and one or plurality of optical devices.

In one example, a light source consists of one or plurality of infra-red light emitting elements is powered by a pulse width modulated input current regulated by the controller.

In one example, a light source consists of one or plurality of infra-red light emitting elements and one or plurality of optical devices.

In one example, a light source consists of one or plurality of infra-red light emitting elements is powered by a pulse width modulated input current regulated by the controller.

In one example, the one or plurality of visible light emitting elements are coupled with the one or plurality of optical devices have the radiation luminous intensity distribution output approachable on an anterior chamber, a posterior chamber, and an eyelid of an eye.

In one example, the one or plurality of infra-red light emitting elements are coupled with the one or plurality of optical devices have the radiation luminous intensity distribution output approachable on an anterior chamber, a posterior chamber, and an eyelid of an eye.

In one example, the optical devices consist of a light guide.

In one example, the optical devices consist of an optical lens.

Although the present invention has been illustrated and described herein with reference to preferred embodiments and specific examples thereof, it will be readily apparent to those of ordinary skill in the art that other embodiments and examples may perform similar functions and/or achieve like results.

It should be understood that the examples and embodiments described herein are for illustrative purposes only and that various modifications or changes in light thereof will be suggested to persons skilled in the art and are to be included within the purview of this application and the scope of the appended claims. In addition, any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

### MATERIALS AND METHODS

The following are examples that illustrate procedures for practicing the invention. These examples should not be construed as limiting. All percentages are by weight and all solvent mixture proportions are by volume unless otherwise noted.

### EXAMPLE 1-

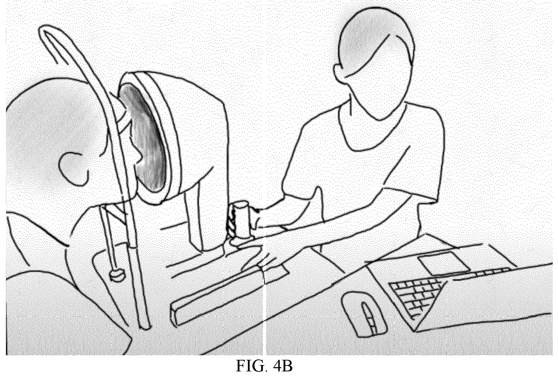

An example of a method [100] for using the present invention to modify the eye condition of a patient is shown in FIG 1. The subject can wear a device in Step [110] that has a temperature-controlled eye pad (e.g., a heat pad) and a pressure chamber covering the periocular region. The heat pad that contacts the eye lid and the periocular skin is set to 40 deg C for 30 minutes of warm compress in Step [120]. After the warm compress, the pressure level of the pressure chamber that covers the periocular region is set to -5 mmHg for 5 minutes, stretching of the ocular tissue in Step [130]. Then the pressure level of the pressure chamber that covers the periocular region is set to -10 mmHg for 5 minutes of further stretching of the ocular tissues in Step [140]. After the execution, the subject can remove the device in Step [150] and repeat the method after a sufficient rest period (e.g., one day.) The experiment is conducted daily at the same hour for a week to investigate the trend of the IOP. Before the IOP measurement, the subject was asked to rest for 5 minutes to calm down. The test apparatus for measurements of IOP is shown in Figure 4. The subjects must place their head on the tonometer holder and their forehead touches the front holder. The tonometer shot an air puff to measure the IOP of the right eye of the subject three times. The measurement was repeated for measurement of the left eye. To verify the effect of the device, a trend of weekly IOP of the right eye and the left eye were shown in FIG. 2A and FIG. 2B respectively to observe the daily differences. The daily IOPs for both eyes show a decreasing trend. The IOP of the left eye was decreased by 6.96% and the right eye was decreased by 3.55%. after using the device for 7 days.

### EXAMPLE 2-

Another example of a method [300] for using the present invention to modify the eye condition of a patient. The subject can wear a device in Step [310] that has a temperature-controlled eye pad (e.g., a heat pad) and a pressure chamber covering the periocular region. The heat pad that contacts the eyelid and the periocular skin is set to 40 deg C of warm compress during the entire process in Step [320]. During the warm compress, the pressure level of the pressure chamber that covers the periocular region is set to -15 mmHg for 15 seconds, stretching of the ocular tissue in Step [330]. Then the pressure level of the pressure chamber is returned to 0 mmHg for 30 seconds in Step [340]. This pressure cycle Step [330] to Step [340] between -15 mmHg to 0 mmHg is repeated twice. After the stretching of the ocular tissue, the eyes are warm compressed for 12 minutes Step [350]. After the warm compress, the pressure level of the pressure chamber that covers the periocular region is set to -15 mmHg for 15 seconds, stretching of the ocular tissue in Step [360]. Then the pressure level of the pressure chamber is returned to 0 mmHg for 30 seconds in Step [370]. This pressure cycle Step [360] to Step [370] between -15 mmHg to 0 mmHg is repeated twice. After the execution, the subject can remove the device in Step [380] and repeat the method after a sufficient rest period (e.g., one day.) The experiment is conducted daily at the same hour for a week to investigate the trend of the IOP. Before the IOP measurement, the subject was asked to rest for 5 minutes to calm down.

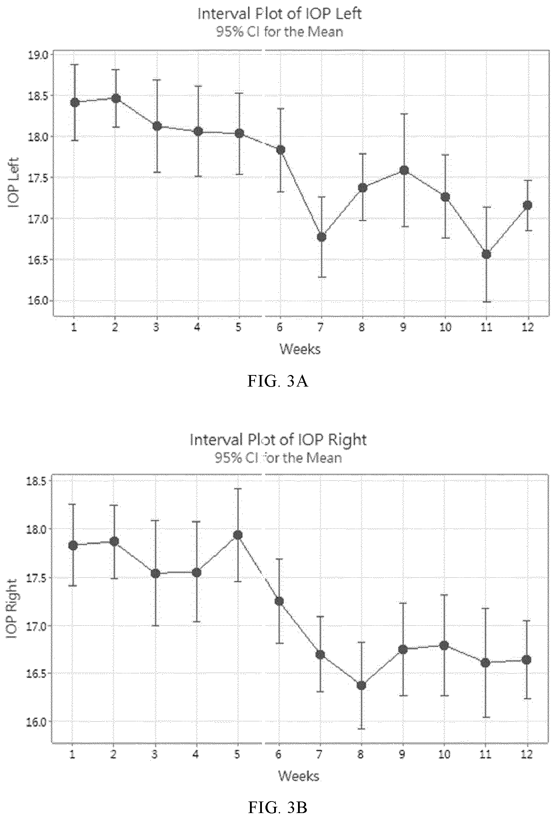

The experimental setup for measurements of IOP is shown in Figure 4. The subjects must place their head on the tonometer holder and their forehead touches the front holder. The tonometer shot an air puff to measure the IOP of the right eye of the subject three times. The measurement was repeated for measurement of the left eye. To verify the effect of the device, a trend of weekly IOP of the right eye and the left eye were shown in FIG. 3A and FIG. 3B respectively to observe the weekly differences for 12 weeks. The IOP data for the first two weeks were the baseline IOP and no device usage during the period and one month prior. The IOP data, starting from week 3 to week 12, are the IOP measurement with the daily usage of the device following method [300].

For one subject, there were 54 measurements for the baseline IOP and 242 for the IOP during the device's usage. The baseline mean average for the left eye and right eye IOP were 18.44 mmHg and 17.85 mmHg respectively. The mean average for left eye and right eye IOP at the end of using the device were 17.45 mmHg and 17.02 mmHg respectively. The decrease in mean IOP for left eye and right eye are 0.99 mmHg and 0.83 mmHg respectively. The respective P-values for comparing the IOP baseline and IOP at the end of device usage for both left eye and right eye were calculated as effectively 0, meaning that the IOP for each eye before and after using the device for 10 weeks were significantly different.

For another subject, there were 66 measurements for the baseline IOP and 276 for the IOP during the device's usage. The baseline mean average for the left eye and right eye IOP were 17.55 mmHg and 17.89 mmHg respectively. The mean average for left eye and right eye IOP at the end of using the device were 17.17 mmHg and 17.64 mmHg respectively. The decrease in mean IOP for left eye and right eye are 0.38 mmHg and 0.25 mmHg respectively. The P-value for comparing the baseline and IOP at the end of device usage for left eye and right eye were 0.031 and 0.136 respectively. Therefore, the IOP of the left eye before and after using the device for 15 weeks were significantly different.

It should be understood that the examples and embodiments described herein are for illustrative purposes only and that various modifications or changes in light thereof will be suggested to persons skilled in the art and are to be included within the purview of this application and the scope of the appended claims. In addition, any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

## Claims

1. A device for improving a state of an eye in a subject, the device comprising a pressure control module configured and adapted to stretch the cornea, the peripapillary sclera, or the extraocular muscle of the eye to an extent sufficient to measurably change a periocular pressure of the eye, or an intraocular pressure of the eye, or both;
wherein the device further comprises:
a goggle carrier;
a heat pad module configured and adapted to warm a cornea, a peripapillary sclera, or an extraocular muscle of the eye to a threshold value that is above a skin surface temperature of the subject;
a radiation module configured and adapted to irradiate one or more tissues of an optic nerve head and the peripapillary sclera in a posterior chamber of the eye with an infra-red radiation having a peak wavelength between 780 nm and 950 nm and with a visible light radiation having a peak wavelength between 550 to 700 nm; and
a controller operably connected to the heat pad module, the pressure control module, and the radiation module, the controller configured and adapted to provide heat, pressure, and radiation until reaching a desired state comprising a measureable change in at least one of reduction of an intraocular pressure of the eye, an increase of a vascular circulation of a peripapillary vessel of the eye, and an increase of a metabolic rate of one or more groups of cells of the eye.

2. The device according to claim 1, wherein the goggle carrier is covering a periocular region of the eye.

3. The device according to claim 1, wherein the eye is a first eye and the goggle carrier is covering the periocular region the first eye and also covering a periocular region of a second eye.

4. The device according to claim 1, wherein the heat pad module comprises a heating element layer, a thermal insulation layer, and a soft contact layer.

5. The device according to claim 4, wherein the heating element layer consists of a resistive heating strip embedded in a flexible printed circuit.

6. The device according to claim 5, wherein the resistive heating strip embedded in the flexible printed circuit is powered by a pulse width modulated input voltage regulated by the controller.

7. The device according to claim 6, wherein the thermal insulation layer is in contact with a first side of the heating element layer.

8. The device according to claim 7, wherein the thermal insulation layer comprises a flexible material that conforms to a curved surface on or around the eye.

9. The device according to claim 8, wherein the soft contact layer is in contact with a second side of the heating element layer.

10. The device according to claim 9, wherein the soft contact layer is configured and adapted for contact with a layer of skin on an eyelid covering the eye.

11. The device according to claim 2, wherein the pressure control module comprises a pump.

12. The device according to claim 11, wherein the pressure control module comprises a first cannula fluidly coupling the pump to a cavity of the goggle carrier adjacent the periocular region.

13. The device according to claim 12, wherein the pressure control module comprises a second cannula fluidly coupling the cavity to an area of ambient pressure.

14. The device according to claim 13, wherein the second cannula comprises a check valve configured and adapted to allow fluid flow from the area of ambient pressure into the cavity while inhibiting fluid flow from the cavity into the area of ambient pressure.

15. The device according to claim 14, wherein the check valve is configured and adapted to open when the gauge pressure in the cavity drops below a threshold pressure.

## Patentansprüche

1. Vorrichtung zum Verbessern eines Zustands eines Auges bei einer Person, wobei die Vorrichtung ein Druckregelmodul umfasst, das dazu konfiguriert und geeignet ist, die Hornhaut, die peripapilläre Lederhaut oder den extraokulären Muskel des Auges so weit zu dehnen, dass ein periokulärer Druck des Auges oder ein intraokulärer Druck des Auges oder beide messbar verändert werden;
wobei die Vorrichtung ferner umfasst:
einen Brillenträger;
ein Heizkissenmodul, das dazu konfiguriert und geeignet ist, eine Hornhaut, eine peripapilläre Lederhaut oder einen extraokulären Muskel des Auges auf einen Schwellenwert, der über einer Hautoberflächentemperatur der Person liegt, zu erwärmen;
ein Strahlungsmodul, das dazu konfiguriert und geeignet ist, ein oder mehrere Gewebe eines Sehnervenkopfes und der peripapillären Lederhaut in einer posterioren Kammer des Auges mit einer Infrarotstrahlung, die eine Spitzenwellenlänge zwischen 780 nm und 950 nm aufweist, und mit einer sichtbaren Lichtstrahlung, die eine Spitzenwellenlänge zwischen 550 bis 700 nm aufweist, zu bestrahlen; und
einen Regler, der betriebsfähig mit dem Heizkissenmodul, dem Druckregelmodul und dem Strahlungsmodul verbunden ist, wobei der Regler dazu konfiguriert und geeignet ist, Wärme, Druck und Strahlung bereitzustellen, bis ein gewünschter Zustand erreicht ist, der eine messbare Veränderung mindestens einer von einer Reduzierung einer eines intraokulären Drucks des Auges, einer Erhöhung einer vaskulären Zirkulation eines peripapillären Gefäßes des Auges und einer Erhöhung einer Stoffwechselrate einer oder mehrerer Gruppen von Zellen des Auges umfasst.

2. Vorrichtung nach Anspruch 1, wobei der Brillenträger eine periokuläre Region des Auges abdeckt.

3. Vorrichtung nach Anspruch 1, wobei das Auge ein erstes Auge ist, und der Brillenträger die periokuläre Region des ersten Auges abdeckt und auch eine periokuläre Region eines zweiten Auges abdeckt.

4. Vorrichtung nach Anspruch 1, wobei das Heizkissenmodul eine Heizelementeschicht, eine Wärmeisolierschicht und eine weiche Kontaktschicht umfasst.

5. Vorrichtung nach Anspruch 4, wobei die Heizelementeschicht aus einem Widerstands-Heizstreifen besteht, der in einer biegsamen gedruckten Schaltung eingebettet ist.

6. Vorrichtung nach Anspruch 5, wobei der Widerstands-Heizstreifen, der in der biegsamen gedruckten Schaltung eingebettet ist, durch eine pulsweitenmodulierte Eingangsspannung, die von dem Regler reguliert wird, mit Energie versorgt wird.

7. Vorrichtung nach Anspruch 6, wobei die Wärmeisolierschicht mit einer ersten Seite der Heizelementeschicht in Kontakt steht.

8. Vorrichtung nach Anspruch 7, wobei die Wärmeisolierschicht ein biegsames Material umfasst, das sich an eine gekrümmte Oberfläche auf dem oder um das Auge herum anpasst.

9. Vorrichtung nach Anspruch 8, wobei die weiche Kontaktschicht mit einer zweiten Seite der Heizelementeschicht in Kontakt steht.

10. Vorrichtung nach Anspruch 9, wobei die weiche Kontaktschicht zum Kontakt mit einer Hautschicht an einem Augenlid, welches das Auge abdeckt, konfiguriert und geeignet ist.

11. Vorrichtung nach Anspruch 2, wobei das Druckregelmodul eine Pumpe umfasst.

12. Vorrichtung nach Anspruch 11, wobei das Druckregelmodul eine erste Kanüle umfasst, welche die Pumpe fluidtechnisch mit einem Hohlraum des Brillenträgers angrenzend an die periokuläre Region koppelt.

13. Vorrichtung nach Anspruch 12, wobei das Druckregelmodul eine zweite Kanüle umfasst, die fluidtechnisch den Hohlraum mit einem Umgebungsdruckbereich koppelt.

14. Vorrichtung nach Anspruch 13, wobei die zweite Kanüle ein Rückschlagventil umfasst, das dazu konfiguriert und geeignet ist, einen Fluidfluss von dem Umgebungsdruckbereich in den Hohlraum zuzulassen, während es einen Fluidfluss von dem Hohlraum in den Umgebungsdruckbereich unterbindet.

15. Vorrichtung nach Anspruch 14, wobei das Rückschlagventil dazu konfiguriert und geeignet ist, sich zu öffnen, wenn der Manometerdruck in dem Hohlraum unter einen Schwellendruck abfällt.

## Revendications

1. Dispositif destiné à améliorer l'état de l'œil d'un sujet, le dispositif comprenant un module de contrôle de pression configuré et adapté pour étirer la cornée, la sclère péripapillaire ou le muscle extraoculaire de l'œil dans une mesure suffisante pour modifier de manière mesurable la pression périoculaire de l'œil, ou la pression intraoculaire de l'œil, ou les deux ;
dans lequel le dispositif comprend en outre :
un support de lunettes ;
un module de coussin chauffant configuré et adapté pour réchauffer une cornée, une sclère péripapillaire ou un muscle extraoculaire de l'œil jusqu'à une valeur seuil supérieure à la température de surface de la peau du sujet ;
un module de rayonnement configuré et adapté pour irradier un ou plusieurs tissus de la tête du nerf optique et de la sclère péripapillaire dans la chambre postérieure de l'œil avec un rayonnement infrarouge ayant une longueur d'onde maximale comprise entre 780 nm et 950 nm et avec un rayonnement de lumière visible ayant une longueur d'onde maximale comprise entre 550 nm et 700 nm ; et
un contrôleur connecté de manière opérationnelle au module de coussin chauffant, au module de contrôle de la pression et au module de rayonnement, le contrôleur étant configuré et adapté pour fournir de la chaleur, de la pression et du rayonnement jusqu'à atteindre un état souhaité comprenant un changement mesurable dans au moins l'un des éléments suivants : une réduction de la pression intraoculaire de l'œil, une augmentation de la circulation vasculaire péripapillaire des vaisseaux de l'œil, et une augmentation du taux métabolique d'un ou plusieurs groupes de cellules de l'œil.

2. Dispositif selon la revendication 1, dans lequel le support de lunettes recouvre une région périoculaire de l'œil.

3. Dispositif selon la revendication 1, dans lequel l'œil est un premier œil et le support de lunettes recouvre la région périoculaire du premier œil ainsi qu'une région périoculaire d'un deuxième œil.

4. Dispositif selon la revendication 1, dans lequel le module de coussin chauffant comprend une couche d'élément chauffant, une couche d'isolation thermique et une couche de contact souple.

5. Dispositif selon la revendication 4, dans lequel la couche d'élément chauffant est constituée d'une bande chauffante résistive intégrée dans un circuit imprimé flexible.

6. Dispositif selon la revendication 5, dans lequel la bande chauffante résistive intégrée dans le circuit imprimé flexible est alimentée par une tension d'entrée modulée en largeur d'impulsion et régulée par le contrôleur.

7. Dispositif selon la revendication 6, dans lequel la couche d'isolation thermique est en contact avec une première face de la couche d'élément chauffant.

8. Dispositif selon la revendication 7, dans lequel la couche d'isolation thermique comprend un matériau souple qui épouse la courbure de la surface de l'œil ou de la zone environnante.

9. Dispositif selon la revendication 8, dans lequel la couche de contact souple est en contact avec une deuxième face de la couche d'élément chauffant.

10. Dispositif selon la revendication 9, dans lequel la couche de contact souple est conçue et adaptée pour être en contact avec une couche de peau de la paupière recouvrant l'œil.

11. Dispositif selon la revendication 2, dans lequel le module de contrôle de pression comprend une pompe.

12. Dispositif selon la revendication 11, dans lequel le module de régulation de pression comprend une première canule reliant de manière fluidique la pompe à une cavité du support de lunettes située à proximité de la région périoculaire.

13. Dispositif selon la revendication 12, dans lequel le module de contrôle de pression comprend une deuxième canule reliant de manière fluidique la cavité à une zone à pression ambiante.

14. Dispositif selon la revendication 13, dans lequel la deuxième canule comprend un clapet anti-retour configuré et adapté pour permettre l'écoulement de fluide de la zone de pression ambiante vers la cavité tout en empêchant l'écoulement de fluide de la cavité vers la zone de pression ambiante.

15. Dispositif selon la revendication 14, dans lequel le clapet anti-retour est configuré et adapté pour s'ouvrir lorsque la pression manométrique dans la cavité chute en dessous d'une pression seuil.
